# EUROPEAN PATENT APPLICATION

(11) **EP 1 844 814 A1**
(43) Veröffentlichungstag der Anmeldung: **17.10.2007**
(21) Anmeldenummer: 06716848.4
(22) Anmeldetag: 24.01.2006
(51) Int. Cl.: A61N 5/02, A61N 5/06, A61N 5/10, A61N 7/00, H01J 13/02, H05H 9/00, A61B 8/00

(54) **PHYSIOTHERAPEUTISCHE VORRICHTUNG**

(30) Priorität: 27.01.2005 RU 2005101941
(71) Anmelder: Kobzev, Anatoly Vassilievich, Tomsk 634034 (RU)
(72) Erfinder: Kobzev, Anatoly Vassilievich, Tomsk 634034 (RU)
(74) Vertreter: Jeck, Anton
(86) Internationale Anmeldenummer: PCT/RU2006/000022
(87) Internationale Veröffentlichungsnummer: WO 2006/083194

(57) **Zusammenfassung**

Die Erfindung bezieht sich auf die Medizin. Die physiotherapeutische Vorrichtung gemäß der Erfindung umfasst eine Einheit zur Erzeugung einer physiotherapeutischen Einwirkung, eine Steuereinheit, eine die Einwirkung lokalisierende Einheit, die mit der die Einwirkung lokalisierenden Fläche verbunden ist, eine feste Basis und eine Einheit zur Verschiebung längs der X-, Y-, Z-Koordinaten, wobei die physiotherapeutische Vorrichtung in Form eines Rahmens, der auf der festen Basis derart befestigt ist, dass er längs linearer X-, Y-, Z-Koordinaten bewegbar ist, und in Form von physiotherapeutischen Wirkungsquellen gebildet ist, die längs des Rahmenumfangs angeordnet sind. Der Rahmen ist mechanisch mit entsprechenden Ausgängen der Einheit zur Verschiebung längs der X-, Y-, Z-Koordinaten verbunden. Die Eingänge dieser Einheit sind an die Ausgänge der Steuereinheit gemäß den X-, Y-, Z-Koordinaten angeschlossen. Die entsprechenden Eingänge der Steuereinheit sind mit den zusätzlichen Ausgängen der Einheit zur Verschiebung längs der X-, Y-, Z-Koordinaten verbunden, und der Steuereingang der Steuereinheit ist mit dem Ausgang der die Einwirkung lokalisierenden Einheit verbunden.

## Beschreibung

Die Erfindung gehört zum Gebiet der Medizin und zwar zu Geräten mit breitem Wirkungsspektrum, das die Vorbeuge- und physiotherapeutische Heilwirkung auf den menschlichen Organismus und andere, biologische Objekte einschließt. Die Erfindung kann für die physiotherapeutische Einwirkung auf pathologische Herde im Organismus Anwendung finden, insbesondere zur Zerstörung von gut- und bösartigen Geschwülsten und anderen konzentrierten Bildungen, wie Steinen in den inneren Organen. Außerdem kann sie zur Stimulierung und/oder Korrektur des funktionalen Zustands des Organismus, zur Vorbeugung verschiedener Krankheiten und für physiotherapeutische Prozeduren verwendet werden, die die Arbeit und Lebenstätigkeit bestimmter Bereiche der inneren Organe und Gewebe stimulieren.

Es sind verschiedene Geräte zur Einwirkung auf die pathologischen Herde in lebenden Organismen weit bekannt, wobei das Wirkungsprinzip dieser Geräte durch Verfahren der physikalischen Einwirkung auf pathologische Herde bestimmt wird; diese Geräte arbeiten beispielsweise mit Ultraschall, Magnetfeldern, Mikrowellenstrahlen, Signalen der Strahlentherapie usw.

Eine breite Anwendung finden in der Medizin technische Mittel zur Strahleneinwirkung, beispielsweise Linearbeschleuniger, Kobaltkanonen usw., auf die Neubildungen in den inneren Organen. Dabei werden die genannten Mittel zur Strahleneinwirkung öfter in Verbindung mit der Einführung von Arzneimitteln, der Chemotherapie usw. verwendet.

Bekannt sind beispielsweise Geräte, die eine unterschiedliche Dosierung der Bestrahlung ermöglichen, je nach den individuellen Werten der Grenzbeschleunigung der Durchblutung im Bestrahlungsbereich (Patent RU Nr. 2088285, A 61 N 5/06, 1997.08.27), unter Berücksichtigung der Verbreitung von Tumorprozessen (Patent RU Nr. 2089247, A 61 N 5/10, 1997.09.10) und je nach anderen Heilmitteln, die dem Kranken gleichzeitig mit der Strahlentherapie verordnet werden (Patent RU Nr. 2163823, A 61 N 5/00, 2001.03.10).

In den genannten Geräten zur Strahleneinwirkung ist aber die Schwere der Strahlenreaktionen und Komplikationen in den den Einwirkungsherd umgebenden Organen und Geweben hoch und die Oberflächendosis im Bestrahlungsbereich maximal.

Bekannt ist ein Gerät zur Mikrowellentherapie (Patent RU Nr. 2048822, A 61 N 1/04, 1995.11.27), das Elektroden mit individuellen Applikatoren umfasst, in denen zur erhöhten Lokalisierung des elektrischen Hochfrequenzfelds im vorgegebenen Körperbereich die Elektroden und die mit diesen verbundenen Applikatorwände beweglich sind.

In diesem Gerät ist aber die Einwirkungsleistung in einem bestimmten Bereich beschränkt, und es ist mit eventuellen Komplikationen in den inneren Organen und den den pathologischen Herd umgebenden Geweben verbunden.

Bekannt sind Geräte zur therapeutischen Einwirkung, die kombinierte Verfahren unter Anwendung der Strahlentherapie realisieren, bei denen die Schwere der allgemeinen, toxischen Reaktion und der Strahlenreaktionen bei der Durchführung der Strahlentherapie berücksichtigt wird. Im Zusammenhang damit wird zur Schwächung der Strahlenschädigung das Arzneimittel auf den Bestrahlungsbereich aufgetragen (Patent RU Nr. 2089247, A 61 N 5/10,1997.09.10).

Diese Geräte erlauben aber ebenso nicht, die Schwere der allgemeinen, toxischen Reaktionen der Patienten ausreichend zu vermindern, die durch die Anwendung der Strahlentherapie in den inneren Organen und Geweben hervorgerufen werden, denn diese bleiben ungeschützt.

Bekannt ist ferner ein Gerät zur Einwirkung auf die an bösartigen Tumoren erkrankten Patienten, das ein Mittel zur Bestrahlung des Tumorherds, eine Einheit für die Volumenbestimmung des Tumorbefalls und dessen regionaler Metastasierungsbereiche und eine Steuereinheit einschließt. Diese erlaubt, die Werte der Strahleneinwirkung zu variieren und die lokale Einwirkung auf die klinisch bestimmten, pathologischen Herde zu steuern (Patent RU Nr. 2141366, A 61 N 5/10, 1997.06.20). Das genannte Gerät erlaubt, das Volumen der Bestrahlung vom minimalen bis zum maximalen Niveau zu variieren, beispielsweise indem der dritte Teil der radikalen, akkumulierten Dosis dem Bereich des pathologischen Herds zugeführt wird. Dabei werden die Komplikationen am Anfang der Bestrahlung vermindert. Ein schroffer Gradient der Dosis zwischen den bestrahlten und unbestrahlten Geweben wird gebildet. Dieses Gerät erlaubt aber nicht, die Beschädigungen in den den pathologischen Herd umgebenden Geweben und inneren Organen wesentlich zu vermindern, falls die radikalen Bestrahlungsdosen zur Erzielung von bestimmten Ergebnissen der Bestrahlungsbeeinflussung notwendig sind.

Bekannt ist weiterhin ein Gerät zur Behandlung der Harnsteinkrankheit (Patent RU Nr. 2125899, A 61 N 5/06, A 61 N 2/04, 1995.06.14), das eine Einheit für die Einwirkung mit Strom mit einer Frequenz von 10-130 Hz und ein Schema für eine kombinierte Magnet-Laser-Einwirkung einschließt.

Das genannte Gerät weist auch Nachteile auf, die den oben angeführten Geräten eigen sind, denn es fehlt die Möglichkeit, die notwendigen Schwellenkonzentrationen der Einwirkung an der lokalisierten Stelle ohne negative Reaktionen seitens der den pathologischen Herd umgebenden Gewebe und Organe zu verwenden.

Nach dem technischen Wesen und erzielten Ergebnis kommt der vorliegenden Erfindung ein Gerät zur Einwirkung auf an bösartigen Tumoren erkrankte Patienten am nächsten (Patent RU Nr. 2128060, A 61 N 5/00, veröff. 1999.03.27), das eine Einheit für die Tumorlokalisierung, eine Steuereinheit und einen Apparat für physiotherapeutische Einwirkung einschließt, der die Einstellung von Sektor-Bestrahlungsfeldem sowie den Abstand von der Quelle bis zum Tumor regelt. Der Apparat für physiotherapeutische Einwirkung umfasst ein Gerät zur Verwirklichung der beweglichen Sektorbestrahlung mit einem Schwingungsbogen, der erlaubt, eine beliebige Anzahl der Bestrahlungen des pathologischen Herds mit einer bestimmten Winkelgeschwindigkeit und einer einmaligen Herddosis durchzuführen. Dabei entspricht die Anzahl der Bestrahlungen der Anzahl der Schwingungen.

Bei diesem Gerät trägt die Verwendung der beweglichen Sektorbestrahlung zur Konzentrierung der Strahleneinwirkung unmittelbar an Tumorherden mit der verminderten Dosenbelastung bei. Aber die Verwendung einer Strahlungsquelle, die durch Schwingungsbögen bewegt wird, in solchen Geräten erlaubt nicht, die notwendige Konzentration der Strahleneinwirkung mit minimalen Strahlenkomplikationen in den umgebenden Geweben optimal zu verbinden.

Die Hauptaufgabe der vorliegenden Erfindung besteht darin, ein Gerät zur physiotherapeutischen Einwirkung zu schaffen, das erlaubt, die Wirksamkeit der Einwirkung bei einer gleichzeitigen Verminderung von negativen Reaktionen auf die physiotherapeutische Einwirkung in den umgebenden Geweben und Organen zu erhöhen.

Die gestellte Aufgabe wird dadurch erfüllt, dass in das bekannte Gerät zur physiotherapeutischen Einwirkung, das einen Apparat für die physiotherapeutische Einwirkung, eine Steuereinheit und eine mit dem Einwirkungsbereich verbundene Einheit für die Lokalisierung der Einwirkung umfasst, eine feste Basis und eine Einheit zur Bewegung in den X-, Y-, Z-Koordinaten eingeführt werden. Außerdem ist der Apparat für die physiotherapeutische Einwirkung als Rahmen ausgebildet, der auf der festen Basis in den X-, Y-, Z-Linearkoordinaten beweglich ist, und mit n Quellen der physiotherapeutischen Einwirkung versehen, die am Umfang des Rahmens angeordnet sind. Der Rahmen ist mechanisch mit entsprechenden Ausgängen der Einheit zur Bewegung in den X-, Y-, Z-Koordinaten verbunden, deren Eingänge an die entsprechenden X-, Y-, Z-Ausgänge der Steuereinheit angeschlossen sind. Die Eingänge der Steuereinheit sind jeweils mit zusätzlichen Ausgängen der Einheit zur Bewegung in den X-, Y-, Z-Koordinaten verbunden. Dabei ist der Steuereingang der Steuereinheit mit dem Ausgang der Einheit für die Lokalisierung der Einwirkung verbunden.

Es ist zweckmäßig, die Anzahl n der Quellen der physiotherapeutischen Einwirkung unter Berücksichtigung der Größe des Einwirkungsherds und der zulässigen Einwirkungsdosis auszuwählen.

Die Quellen der physiotherapeutischen Einwirkung können als Mini-Elektronenkanonen oder Ultrahochfrequenzgeneratoren (UHF-Generatoren) oder L-Generatoren oder Ultraschallgeneratoren oder andere Quellen der physiotherapeutischen Einwirkung je nach der erforderlichen Einwirkung ausgebildet werden.

Außerdem ist es möglich, die Quellen der physiotherapeutischen Einwirkung in jeder Kombination einzusetzen, falls beispielsweise kombinierte Einwirkungsverfahren zur Anwendung kommen.

Die Art der Quelle der physiotherapeutischen Einwirkung wird in Abhängigkeit von der erforderlichen Einwirkung auf den pathologischen Herd oder von der Stimulierung des funktionalen Zustands des Organismus gewählt. Falls die Neubildungen zerstört werden müssen, können die Quellen der radioaktiven Bestrahlung oder Röntgenbestrahlung verwendet werden. Falls die thermische Einwirkung auf die Neubildung als Hauptfaktor bei der Einwirkung verwendet wird, können die Strahler in Form von Mikrowellengeneratoren im Bereich von 1-10 GHz oder Mini-Elektronenkanonen eingesetzt werden. Im Falle der Einwirkung auf die konzentrierten Bildungen wie Steine in den inneren Organen können Ultraschallgeneratoren verwendet werden.

Es ist zweckmäßig, den Rahmen als Ring oder Halbkugel auszuführen, der bzw. die den Oberteil der festen Basis umfasst.

Die feste Basis kann gelocht ausgeführt werden, um die Einwirkung am ganzen Umfang des Rahmens durchzuführen.

Es ist zweckmäßig, auf dem Ring ausziehbare Stangen zu installieren; auf jeder dieser Stangen kann eine Quelle zur physiotherapeutischen Einwirkung montiert werden.

Dabei sollen die Quellen der physiotherapeutischen Einwirkung auf dem Ring oder den Teleskopstangen auf solche Weise montiert werden, dass die von ihnen ausgehenden Einwirkungssignale einen Konus bilden. Als Basis des Konus dient die Fläche des Rings. Die Konusspitze ist der Kreuzungspunkt der Strahlen oder Signale.

Die Steuereinheit kann als Speichereinheit für die X-, Y-, Z-Koordinaten, Bearbeitungseinheit für die Signale der X-, Y-, Z-Koordinaten-Sensoren, Vergleichseinheit für die X-, Y-, Z-Koordinaten und Verstärkereinheit ausgeführt werden. Dabei ist die Gruppe der Ausgänge bei der Speichereinheit für die X-, Y-, Z-Koordinaten mit den entsprechenden, direkten Eingängen der Vergleichseinheit für die X-, Y-, Z-Koordinaten verbunden. Die Gruppe der Eingänge der Bearbeitungseinheit für Signale der Koordinatensensoren stellt dementsprechend die Gruppe der Eingänge der Steuereinheit dar, und die Gruppe der Ausgänge der Bearbeitungseinheit für die Signale der Koordinatensensoren ist mit den entsprechenden, inversen Eingängen der Vergleichseinheit für die X-, Y-, Z-Koordinaten verbunden. Dabei sind die Ausgänge der Vergleichseinheit für die X-, Y-, Z-Koordinaten mit den entsprechenden Eingängen der Verstärkereinheit verbunden, deren Ausgänge diejenigen für die X-, Y-, Z-Koordinaten der Steuereinheit darstellen. Der Eingang der Speichereinheit für die X-, Y-, Z-Koordinaten ist der Steuereingang der Steuereinheit.

Es ist zweckmäßig, die Vergleichseinheit als Vergleichsschaltung nach der X-Koordinate, eine Vergleichsschaltung nach der Y-Koordinate und eine Vergleichsschaltung nach der Z-Koordinate und die Verstärkereinheit mit einem ersten, zweiten und dritten Verstärker auszuführen, dessen Eingang jeweils mit dem entsprechenden Ausgang der Vergleichsschaltungen verbunden ist.

Die Einheit zur Bewegung in den X-, Y-, Z-Koordinaten kann als für jede Koordinate reihengeschalteter Elektromotor, Koordinatensensor des Rahmens, dessen erster Ausgang mit dem Getriebe verbunden ist, und Schraubengetriebe ausgeführt werden. Dabei stellen die Eingänge der Elektromotoren für jede der Koordinaten die entsprechenden Eingänge der Einheit zur Bewegung in den X-, Y-, Z-Koordinaten dar. Die Ausgänge der Schraubengetriebe für jede der Koordinaten sind die entsprechenden Ausgänge der Einheit zur Bewegung in den X-, Y-, Z-Koordinaten, und die zweiten Ausgänge der Koordinatensensoren des Rahmens sind die entsprechenden, zusätzlichen Ausgänge der Einheit zur Bewegung in den X-, Y-, Z-Koordinaten.

Falls die Strahleneinwirkung durchgeführt wird, ist es zweckmäßig, das Gerät mit einem zusätzlichen Ring zur Absorption der Strahlen auszurüsten, die den Einwirkungsbereich durchstrahlen.

Die verteilte, physiotherapeutische Oberflächen- und Innengewebeeinwirkung, die durch die Ausführung des Einwirkungsapparats mit n Quellen erzielt wird, und die Ausführung des Geräteaufbaus derart, dass die Signale der Einwirkungsquellen anfangs im Kreuzungspunkt zusammengeführt werden, der danach am Aufenthaltsort des mit den Linearkoordinaten x, y, z lokalisierten, pathologischen Herds oder anderen Einwirkungsbereichs angesetzt wird, erlaubt, die physiotherapeutische Einwirkung mit der erforderlichen Leistung unmittelbar an der Lokalisierungsstelle der Einwirkung mit einer minimalen Dosisbelastung der den Einwirkungsbereich umgebenden Organe und Gewebe zu konzentrieren.

Das Gerät zur physiotherapeutischen Einwirkung ist als Rahmen mit darauf verteilten, n Quellen der physiotherapeutischen Einwirkung ausgeführt. Die Quellen sind in der X-Richtung, Y-Richtung und Z-Richtung in den existierenden X-, Y-, Z-Koordinaten beweglich. Das Gerät zur physiotherapeutischen Einwirkung ist auf einer festen Basis montiert, auf der innerhalb des Rings der Patient untergebracht wird. Eine derartige Ausführung des Geräts sowie die Steuereinheit, die mit der Einheit zur Bewegung des Rings in den X-, Y-, Z-Koordinaten verbunden ist, erlaubt, die Wirksamkeit der Einwirkung zu steigern, wobei ein maximales Signal in einer bestimmten Einwirkungszone konzentriert wird. Dabei wird durch diese physiotherapeutische Einwirkung unter Berücksichtigung der am Rahmenumfang (oder am Bogen, an der Ringhälfte) verteilten, n Einwirkungsquellen, deren Strahlen oder Signale in einem Bereich nach den bestimmten X-, Y-, Z-Koordinaten zusammengeführt werden, die Oberflächen- sowie Innengewebsdosis der Einwirkung auf die inneren Organe minimiert.

Die Erfindung wird nun anhand von Ausführungsbeispielen näher erläutert. Es zeigen:
- Fig. 1: eine perspektivische, schematische Gesamtansicht des Aufbaus eines Geräts gemäß der Erfindung und ein zugehöriges Flussschema,
- Fig. 2: eine perspektivische, schematischen Ansicht des Aufbaus nach Fig. 1 und ein Wirkungsschema des Geräts,

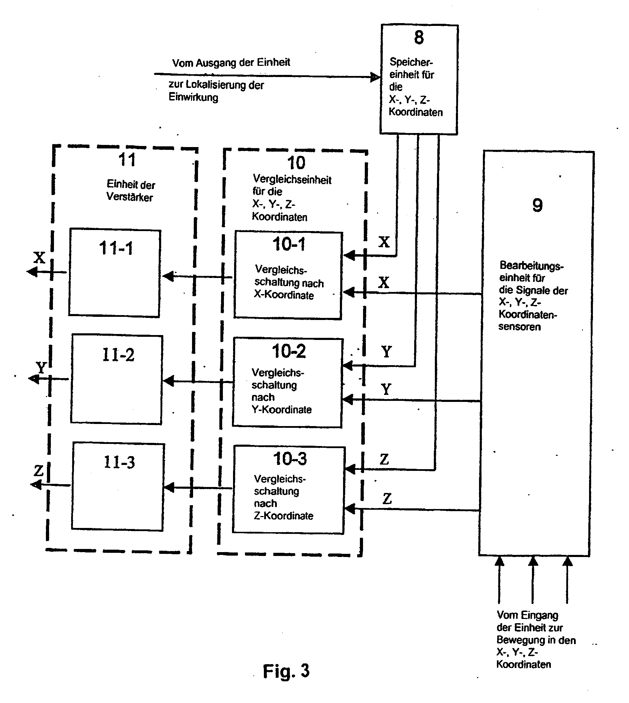

- Fig. 3: ein Wirkungsschema der Steuereinheit der Figuren 1 und 2,

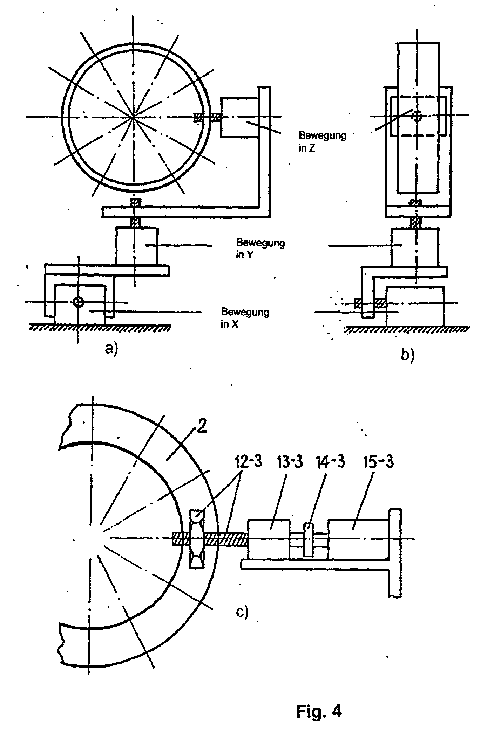

- Fig. 4 (a, b, c): schematische Ansichten des Aufbaus der Einheit zur Bewegung in den X-, Y-, Z-Koordinaten und der Bewegungsmechanik dieser Einheit und

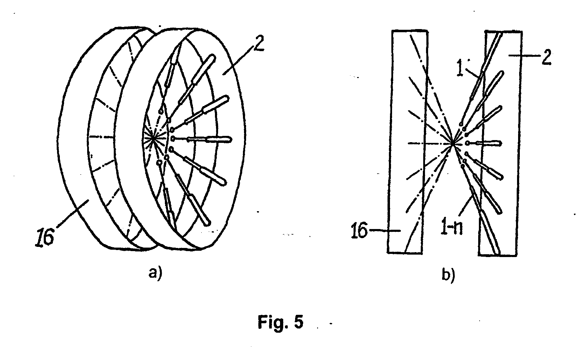

- Fig. 5: schematische Ansichten des Rahmenaufbaus mit ausziehbaren Stangen (Fig. 5a) und einem Absorptionsring (Fig. 5b).

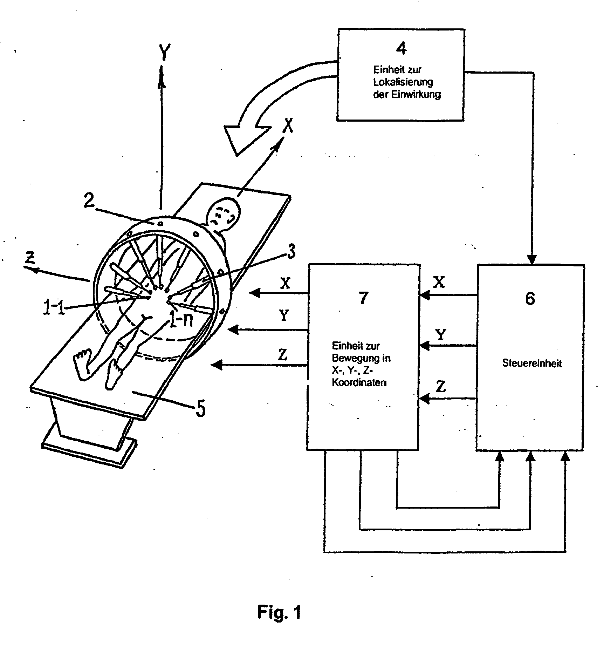

Das Gerät (Fig. 1) enthält einen Apparat zur physiotherapeutischen Einwirkung, der mit n Quellen 1 der physiotherapeutischen Einwirkung ausgeführt ist, die gleichmäßig am Umfang des Rahmens 2 verteilt sind, der ringförmig auf ausziehbaren Stangen 3 angeordnet ist, und eine Einheit 4 zur Lokalisierung der Einwirkung, wobei diese Einheit optisch mit dem Lokalisierungsbereich der Einwirkung verbunden ist. Der Ring 2 ist auf einer festen Basis 5 montiert und ist in der X-Richtung (X-Koordinate), Y-Richtung (Y-Koordinate) und Z-Richtung (Z-Koordinate) beweglich. Das Gerät umfasst auch eine Steuereinheit 6, die mit einer Einheit 7 zur Bewegung in den X-, Y-, Z-Koordinaten verbunden ist, deren Gruppe der Ausgänge mechanisch mit dem Ring 2 verbunden ist.

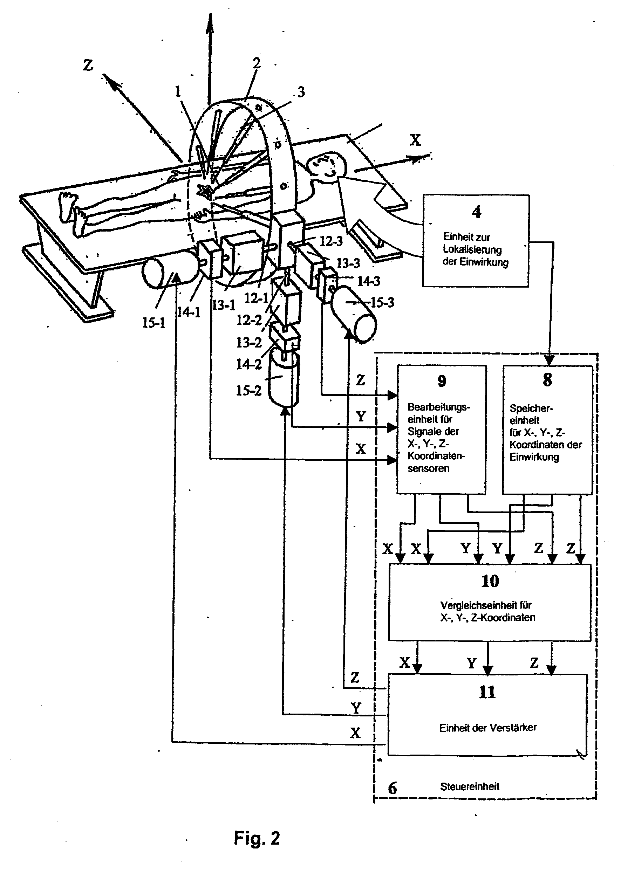

Die Steuereinheit 6 (Fig. 2) umfasst eine Speichereinheit 8 für die X-, Y-, Z-Koordinaten, eine Bearbeitungseinheit 9 für die Signale der X-, Y-, Z-Koordinaten-Sensoren, eine Vergleichseinheit 10, die als Vergleichsschaltung 10-1 in Bezug auf die X-Koordinate, als Vergleichsschaltung 10-2 in Bezug auf die Y-Koordinate, als Vergleichsschaltung 10-3 in Bezug auf die Z-Koordinate ausgeführt ist, und eine Verstärkereinheit 11, die als erster Verstärker 11-1, zweiter Verstärker 11-2 und dritter Verstärker 11-3 ausgeführt ist. Dabei sind die Ausgänge der Speichereinheit 8 für die X-, Y-, Z-Koordinaten jeweils mit den direkten Eingängen X, Y, Z der entsprechenden Vergleichsschaltungen 10-1, 10-2, 10-3 in Bezug auf die X-, Y-, Z-Koordinaten verbunden.

Die Gruppe der Eingänge der Bearbeitungseinheit 9 für die Signale der X-, Y-, Z-Koordinaten-Sensoren stellt die entsprechende Gruppe der Steuereinheit 6 dar, und die Gruppe der Ausgänge der Bearbeitungseinheit 9 für die Signale der X-, Y-, Z-Koordinaten-Sensoren ist mit den entsprechenden, inversen Eingängen X, Y, Z der Vergleichsschaltungen 10-1, 10-2, 10-3 verbunden. Der Ausgang der Vergleichsschaltung 10-1 für die X-Koordinate ist mit dem Eingang des ersten Verstärkers 11-1 verbunden, dessen Ausgang den Ausgang X der Steuereinheit 6 darstellt. Der Ausgang der Vergleichsschaltung 10-2 für die Y-Koordinate ist mit dem Eingang des zweiten Verstärkers 11-2 verbunden, dessen Ausgang den Ausgang Y der Steuereinheit 6 darstellt. Der Ausgang der Vergleichsschaltung 10-3 für die Z-Koordinate ist mit dem Eingang des dritten Verstärkers 11-3 verbunden, dessen Ausgang den Ausgang Z der Steuereinheit 6 darstellt. Dabei stellt der Eingang der Speichereinheit 8 für die X-, Y-, Z-Koordinaten den Steuereingang der Steuereinheit 6 dar.

Die Einheit 7 zur Bewegung (Fig. 2) in den X-, Y-, Z-Koordinaten ist als erstes Schraubengetriebe 12-1, zweites Schraubengetriebe 12-2 und drittes Schraubengetriebe 12-3, erstes Getriebe 13-1, zweites Getriebe 13-2 und drittes Getriebe 13-3, erster Koordinatensensor 14-1, zweiter Koordinatensensor 14-2 und dritter Koordinatensensor 14-3 des Rahmens 2, erster Elektromotor 15-1, zweiter Elektromotor 15-2 und dritter Elektromotor 15-3 ausgeführt, deren Eingänge jeweils die Eingänge X, Y, Z der Einheit 7 zur Bewegung in den Koordinaten X, Y, Z darstellen. Die Ausgänge der Elektromotoren 15-1, 15-2, 15-3 sind jeweils mit den Eingängen des ersten Koordinatensensors 14-1, zweiten Koordinatensensors 14-2 und dritten Koordinatensensors 14-3 des Rahmens 2 verbunden. Die ersten Ausgänge der Sensoren sind jeweils an das erste Getriebe 13-1, zweite Getriebe 13-2 und dritte Getriebe 13-3 angeschlossen. Die zweiten Ausgänge der Koordinatensensoren 14-1, 14-2, 14-3 des Rahmens 2 stellen jeweils zusätzliche Ausgänge der Einheit 7 zur Bewegung in den X-, Y-, Z-Koordinaten dar.

Die n Quellen 1 der physiotherapeutischen Einwirkung können je nach der erforderlichen Einwirkungsdosis und der verordneten Heil- bzw. Vorbeugungswirkung als Mini-Efektronenkanonen, Ultrahochfrequenz-(UHF)-Generatoren, Ultraschallgeneratoren usw. ausgeführt werden.

Die n Quellen 1 der physiotherapeutischen Einwirkung werden vor dem Einsatz des Geräts auf dem Ring 2 oder den ausziehbaren Stangen 3 derart eingerichtet, dass die von ihnen ausgehenden Einwirkungssignale einen Konus bilden. Als Basis des Konus dient die Fläche des Rings 2, wobei die Konusspitze der Kreuzungspunkt der Strahlen oder Signale ist.

Die feste Basis 5 wird gelocht ausgeführt, falls die Einwirkung durch die Quellen durchgeführt wird, die am ganzen Umfang des Rahmens montiert sind, der in Form von einem die feste Basis umfassenden Ring 2 ausgeführt ist. Der Ring 2 kann als Halbkugel ausgeführt werden, die den Oberteil der festen Basis umfasst. Die feste Basis 5 kann dabei als Vollplatte ausgeführt sein.

Bei der Durchführung der Strahleneinwirkung ist das Gerät mit einem zusätzlichen Ring 16 zur Absorption der Strahlen ausgestattet, die die Neubildung oder einen anderen, pathologischen Herd des Patienten durchstrahlen.

Das Gerät zur physiotherapeutischen Einwirkung arbeitet folgendermaßen.

Der Rahmen, der in Form eines Rings 2 ausgeführt ist, wird auf der festen Basis 5 beweglich in der X-Richtung, Y-Richtung und Z-Richtung (X-, Y-, Z-Koordinaten) montiert. Der Ring 2 ist mechanisch mit den festen Schraubengetrieben 12 verbunden, die in Reihe mit den Getrieben 13, Koordinatensensoren 14 des Rahmens und den Elektromotoren verbunden sind. Zur höheren Präzision der Bewegung in den entsprechenden X-, Y-, Z-Linearkoordinaten werden Schrittmotoren bzw. berührungslose Gleichstrommotoren eingesetzt.

Der Ring 2 kann mit Hilfe des festen Schraubengetriebes 12-1, Getriebes 13-1, Koordinatensensors 14-1 des Rahmens, Elektromotors 15-1, die an der festen Basis 5 fest befestigt werden, eine Bewegung an der festen Basis 5 entlang in der X-Koordinate ausführen. Dementsprechend wird mit Hilfe des Schraubengetriebes 12-2, Getriebes 13-2, Koordinatensensors 14-2 des Rahmens und des Elektromotors 15-2 die Bewegung des Rings in der Y-Linearkoordinate ausgeführt. Auf ähnliche Weise wird die Bewegung des Rings 2 in der Z-Linearkoordinate mit Hilfe des Schraubengetriebes 12-3, Getriebes 13-3, Koordinatensensors 14-3 des Rahmens und des Elektromotors 15-3 ausgeführt. Der Patient wird auf der festen Basis innerhalb des Rings 2 untergebracht.

Mit Hilfe der Einheit 4 zur Lokalisierung der Einwirkung wird die Einwirkungszone, nämlich der pathologische Herd oder die Zone der Stimulierung des funktionalen Zustands des inneren Organs oder Gewebes in den X-, Y-, Z-Koordinaten, ermittelt. Die Einheit zur Lokalisierung der Einwirkung kann als Computer-Ultraschalltomograf ausgeführt werden, der die Informationen über den pathologischen Herd oder einen anderen Einwirkungsbereich der drei X-, Y-, Z-Linearkoordinaten erfasst und in die Steuereinheit eingibt.

Bei Einschaltung der Speisespannung des Geräts beginnen die Elektromotoren 15 zu drehen. Dadurch bewegt sich der Ring 2 in allen drei X-, Y-, Z-Koordinaten. Dabei kommen die Signale von den Ausgängen der Koordinatensensoren 14 des Rahmens, die die zusätzlichen Ausgänge der Einheit 7 zur Bewegung in den X-, Y-, Z-Koordinaten darstellen, an die Eingänge der Steuereinheit 6, die die entsprechenden X-, Y-, Z-Eingänge der Bearbeitungseinheit für die Signale der X-, Y-, Z-Koordinaten-Sensoren darstellen.

Der beim Patienten vorermittelte, pathologische Herd oder die mittels der Einheit 4 zur Lokalisierung der Einwirkung in den X-, Y-, Z-Koordinaten erfasste Zone der erforderlichen Einwirkung zur Stimulierung des bestimmten Organs kommt zwecks Organisierung der erforderlichen, physiotherapeutischen Prozedur an den Steuereingang der Steuereinheit 6, der den Eingang der Speichereinheit 8 für die X-, Y-, Z-Koordinaten darstellt. Die Speichereinheit 8 für die X-, Y-, Z-Koordinaten enthält mindestens drei Speicherzellen für X, Y, Z, von deren Ausgang den bestimmten X-, Y-, Z-Koordinaten entsprechende Signale im Digitalkode an die entsprechenden, direkten Eingänge der Vergleichsschaltungen nach X (10-3), nach Y (10-2) und nach Z (10-1) gelangen.

An die inversen Eingänge der entsprechenden Vergleichsschaltungen 10 nach X, Y, Z gelangen die Signale über die X-, Y-, Z-Koordinaten von den entsprechenden Ausgängen der Bearbeitungseinheit 9 für die Signale der X-, Y-, Z-Koordinaten-Sensoren, die der Größe der X-, Y-, Z-Koordinaten proportionalen Punkte der Strahlenzusammenführung, die im Ausgangszustand gleich Null sind. An den Ausgängen der Vergleichseinheit 10 für die X-, Y-, Z-Koordinaten werden Signale gebildet, die proportional der Signaldifferenz der Speichereinheit 8 für die X-, Y-, Z-Koordinaten und der Bearbeitungseinheit 9 für die Signale der X-, Y-, Z-Koordinaten-Sensoren sind. Bei der Übereinstimmung des Signalpegels an den direkten und inversen Eingängen jeder der Vergleichsschaltungen in der Vergleichseinheit 10 werden die Signale an deren Eingängen gleich Null, wobei die Elektromotoren 15 der Einheit 7 zur Bewegung in den X-, Y-, Z-Koordinaten ausgeschaltet werden.

Die Ausschaltung des mechanischen Schaltungssystems der Einheit 7 zur Bewegung in den X-, Y-, Z-Koordinaten bedeutet, dass der Bereich des pathologischen Herds bzw. des Herds der Vorbeugungseinwirkung beim Patienten gemäß den X-, Y-, Z-Linearkoordinaten mit dem Bereich der Zusammenführung der Strahlen oder anderen, physiotherapeutischen Signale zusammenfällt. Die n Quellen 1 der physiotherapeutischen Einwirkung sind auf den ausziehbaren Stangen derart angeordnet, dass die von ihnen ausgehenden Einwirkungssignale einen Konus bilden. Als Basis des Konus dient die Fläche des Rings, wobei die Konusspitze der Kreuzungspunkt der Strahlen oder anderen Signale der physiotherapeutischen Einwirkung ist.

Danach werden automatisch bzw. manuell durch eine Bedienungsperson die ausziehbaren Stangen 3 ausgefahren, auf denen die n Einwirkungsquellen 1 eingebaut sind.

Der zusätzliche Ring 16 ist mit dem Ring 2 fest verbunden und dient zur Absorption der Signale der Strahleneinwirkung, die nach dem Kreuzungspunkt der Strahlen auseinander gehen.

Falls der Rahmen in Form eines Rings 2 ausgeführt ist und die feste Basis umfasst, wird diese gelocht ausgeführt.

Im Gerät kann eine kombinierte Einwirkung auf die pathologischen Herde oder anderen Einwirkungszonen verwendet werden. Im Zusammenhang damit können auf den ausziehbaren Stangen 3 gleichzeitig beispielsweise die Quellen der Strahleneinwirkung und Mikrowellen bzw. in einer anderen Verbindung installiert werden.

Möglich ist auch die Bewegung der festen Basis bei einem unbeweglichen Ringrahmen 2 in den X-, Y-, Z-Richtungen.

Angeschlossen werden die n Quellen 1 der physiotherapeutischen Einwirkung nach der Installation der vorher berechneten Einwirkungsbetriebsarten, die durch die Einwirkungsintensität und -zeit charakterisiert sind. Die Einwirkungsbetriebsarten werden je nach der Größe des pathologischen oder anderen Einwirkungsherds und den gewählten, physikalischen Einwirkungsverfahren eingestellt.

Das Gerät gemäß der Erfindung erlaubt, ein breites Spektrum der physiotherapeutischen Einwirkung auf verschiedene Arten der Neubildungen und anderen, konzentrierten.Bildungen in den inneren Organen auszuführen.

Das Gerät erlaubt, den Aufgabenbereich der Einwirkung von der Stimulierung und Aktivierung der Tätigkeit von gesunden Zellen bis zur Zerstörung von Tumorzellen zu variieren.

Das Gerät erlaubt, auf die pathologischen Herde oder anderen Zonen, die die Vorbeugungs- bzw. Stimulierungseinwirkung benötigen, zweckmäßig und wirksam einzuwirken, wobei die negativen Reaktionen auf die den Einwirkungsbereich umgebenden Organe und Gewebe minimiert werden.

## Patentansprüche

1. Gerät zur physiotherapeutischen Einwirkung, das einen Apparat für die physiotherapeutische Einwirkung, eine Steuereinheit und eine mit dem Einwirkungsbereich verbundene Einheit für die Lokalisierung der Einwirkung umfasst,
**dadurch gekennzeichnet,**
**dass** in dieses Gerät eine feste Basis und eine Einheit zur Bewegung in den X-, Y-, Z-Koordinaten eingeführt ist, dass der Apparat zur physiotherapeutischen Einwirkung als Rahmen ausgebildet ist, der auf der festen Basis in den X-, Y-, Z-Linearkoordinaten beweglich ist, und dass n Quellen der physiotherapeutischen Einwirkung am Umfang des Rahmens angeordnet sind, wobei der Rahmen mechanisch mit entsprechenden Ausgängen der Einheit zur Bewegung in den X-, Y-, Z-Koordinaten verbunden ist, deren Eingänge an die entsprechenden X-, Y-, Z-Ausgänge der Steuereinheit angeschlossen sind, die Eingänge der Steuereinheit jeweils mit zusätzlichen Ausgängen der Einheit zur Bewegung in den X-, Y-, Z-Koordinaten verbunden sind und der Steuereingang der Steuereinheit mit dem Ausgang der Einheit für die Lokalisierung der Einwirkung verbunden ist.

2. Gerät nach Anspruch 1,
**dadurch gekennzeichnet,**
**dass** die Anzahl n der Quellen der physiotherapeutischen Einwirkung unter Berücksichtigung der Größe des Einwirkungsherds und der zulässigen Einwirkungsdosis ausgewählt wird.

3. Gerät nach Anspruch 1,
**dadurch gekennzeichnet,**
**dass** jede der Quellen der physiotherapeutischen Einwirkung als Mini-Elektronenkanone ausgeführt ist.

4. Gerät nach Anspruch 1,
**dadurch gekennzeichnet,**
**dass** jede der Quellen der physiotherapeutischen Einwirkung als UHF-Generator ausgeführt ist.

5. Gerät nach Anspruch 1,
**dadurch gekennzeichnet,**
**dass** jede der Quellen der physiotherapeutischen Einwirkung als L-Generator ausgeführt ist.

6. Gerät nach Anspruch 1,
**dadurch gekennzeichnet,**
**dass** jede der Quellen der physiotherapeutischen Einwirkung als Ultraschallgenerator ausgeführt ist.

7. Gerät nach Anspruch 1,
**dadurch gekennzeichnet,**
**dass** der Rahmen als Ring ausgeführt ist.

8. Gerät nach Anspruch 1,
**dadurch gekennzeichnet,**
**dass** der Rahmen als Halbkugel ausgeführt ist, die den Oberteil der festen Basis umfasst.

9. Gerät nach Anspruch 1,
**dadurch gekennzeichnet,**
**dass** die Basis gelocht ausgeführt ist.

10. Gerät nach Anspruch 7,
**dadurch gekennzeichnet,**
**dass** der Ring mit ausziehbaren Stangen versehen ist, auf denen jeweils eine Quelle der physiotherapeutischen Einwirkung angeordnet ist.

11. Gerät nach Anspruch 10,
**dadurch gekennzeichnet,**
**dass** die Stangen als Teleskopstangen ausgebildet sind.

12. Gerät nach Anspruch 11,
**dadurch gekennzeichnet,**
**dass** die Quellen der physiotherapeutischen Einwirkung auf Teleskopstangen derart angeordnet sind, dass die von ihnen ausgehenden Einwirkungssignale einen Konus bilden, und dass als Basis des Konus die Fläche des Rings dient, wobei die Konusspitze der Kreuzungspunkt der Strahlen oder Signale ist.

13. Gerät nach Anspruch 1,
**dadurch gekennzeichnet,**
**dass** die Steuereinheit eine Speichereinheit für die X-, Y-, Z-Koordinaten, eine Bearbeitungseinheit für die Signale der X-, Y-, Z-Koordinaten-Sensoren, eine Vergleichseinheit für die X-, Y-, Z-Koordinaten und eine Verstärkereinheit enthält, wobei die Gruppe der Ausgänge bei der Speichereinheit für die X-, Y-, Z-Koordinaten mit den entsprechenden, direkten Eingängen der Vergleichseinheit für die X-, Y-, Z-Koordinaten verbunden ist, die Gruppe der Eingänge der Bearbeitungseinheit für Signale der Koordinatensensoren dementsprechend die Gruppe der Eingänge der Steuereinheit darstellt, die Gruppe der Ausgänge der Bearbeitungseinheit für die Signale der Koordinatensensoren mit den entsprechenden, inversen Eingängen der Vergleichseinheit für die X-, Y-, Z-Koordinaten verbunden ist, die Ausgänge der Vergleichseinheit für die X-, Y-, Z-Koordinaten mit den entsprechenden Eingängen der Einheit der Verstärker verbunden sind, deren Ausgänge die für die X-, Y-, Z-Koordinaten der Steuereinheit darstellen, und der Eingang der Speichereinheit für die X-, Y-, Z-Koordinaten der Steuereingang der Steuereinheit ist.

14. Gerät nach Anspruch 13,
**dadurch gekennzeichnet,**
**dass** die Vergleichseinheit eine Vergleichsschaltung in Bezug auf die X-Koordinate, eine Vergleichsschaltung in Bezug auf die Y-Koordinate und eine Vergleichsschaltung in Bezug auf die Z-Koordinate enthält, und jede dieser Schaltungen mit direkten und inversen Ausgängen versehen ist, die dementsprechend direkte und inverse Eingänge der Vergleichseinheit in Bezug auf die X-, Y-, Z-Koordinaten sind.

15. Gerät nach Anspruch 13,
**dadurch gekennzeichnet,**
**dass** die Verstärkereinheit einen ersten, zweiten und dritten Verstärker enthält.

16. Gerät nach Anspruch 1,
**dadurch gekennzeichnet,**
**dass** die Einheit zur Bewegung in den X-, Y-, Z-Koordinaten als für jede Koordinate reihengeschalteter Elektromotor, Koordinatensensor des Rahmens, dessen erster Ausgang mit dem Getriebe verbunden ist, und Schraubengetriebe ausgeführt ist, wobei die Eingänge der Elektromotoren für jede der Koordinaten die entsprechenden Eingänge der Einheit zur Bewegung in den X-, Y-, Z-Koordinaten darstellen, die Ausgänge der Schraubengetriebe für jede der Koordinaten die entsprechenden Ausgänge der Einheit zur Bewegung in den X-, Y-, Z-Koordinaten sind und die zweiten Ausgänge der Koordinatensensoren des Rahmens die entsprechenden, zusätzlichen Ausgänge der Einheit zur Bewegung in den X-, Y-, Z-Koordinaten sind.

17. Gerät nach Anspruch 1,
**dadurch gekennzeichnet,**
**dass** es mit einem zusätzlichen Ring zur Absorption der Strahlen ausgerüstet ist, die den Einwirkungsbereich durchstrahlen.
